(19) Europäisches Patentamt
European Patent Office
Office européen des brevets

(11) Veröffentlichungsnummer : **0 062 005**
**B1**

(12) **EUROPÄISCHE PATENTSCHRIFT**

(45) Veröffentlichungstag der Patentschrift : **26.06.85**

(51) Int. Cl.⁴ : **C 07 C121/70**, C 07 C120/00, C 07 C 15/52, C 07 C 15/46

(21) Anmeldenummer : **82810134.5**

(22) Anmeldetag : **25.03.82**

(54) **Verfahren zur Herstellung von Styrol- und/oder Stilbenderivaten.**

(30) Priorität : **31.03.81 CH 2179/81**

(43) Veröffentlichungstag der Anmeldung : **06.10.82 Patentblatt 82/40**

(45) Bekanntmachung des Hinweises auf die Patenterteilung : **26.06.85 Patentblatt 85/26**

(84) Benannte Vertragsstaaten : **CH DE FR GB IT LI**

(56) Entgegenhaltungen :
**CH-A- 586 644**
**DE-A- 2 602 750**
**DE-B- 1 691 722**
**DE-B- 1 914 601**

(73) Patentinhaber : **CIBA-GEIGY AG**
**Postfach**
**CH-4002 Basel (CH)**

(72) Erfinder : **Spencer, Alwyn, Dr.**
**Schützengraben 15**
**CH-4051 Basel (CH)**

**EP 0 062 005 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von Styrol- und/oder Stilbenderivaten.

Es ist bekannt, dass man vinylisch oder allylisch substituierte organische Verbindungen, u. a. auch Styrole und/oder Stilbene, durch katalytische Umsetzung von entsprechenden Halogeniden mit Olefinen, z. B. Acrylsäuremethylester oder Aethylen, in Gegenwart von tertiären Aminen herstellen kann. Als Katalysatoren werden bevorzugt Gemische von Palladiumacetat und Triphenylphosphin oder Tri-o-tolylphosphin verwendet. Die Umsetzung kann auch so vorgenommen werden, dass man zuerst aus dem Halogenid und dem Katalysatorysystem einen Komplex bildet und diesen anschliessend in Gegenwart eines tertiären Amins mit dem Olefin reagieren lässt. Die Umsetzung von Halogenbenzolen mit Aethylen wird unter Druck, bevorzugt einem Aethylendruck von etwa 1,4 bis 14 bar, durchgeführt, wobei je nach Reaktionsbedingungen und/oder Ausgangs-Halogenbenzol Styrole und/oder Stilbene entstehen [vgl. z. B. U.S. Patentschrift 3.922.299 und J. Org. Chem., *43*, 2454 und *43*, 2941 (1978)].

Gemäss Bull. Chem. Soc. Japan, *46*, 1505 (1973) und CH-AS-586644 lassen sich verschiedene Olefine, u. a. Aethylen oder Propylen, in Gegenwart von Palladiumschwarz oder $PdCl_2$ und eines Ueberschusses an Kalium- bzw. Natriumacetat als Säureakzeptor mit Halogenbenzolen, besonders Jodbenzolen, arylieren. Bei der Umsetzung von Jodbenzol mit Aethylen unter Druck gemäss Bull. Chem. Soc. Japan, *46*, 1505 (1973) wird dabei mit einer Selektivität von 60-90 % Styrol gebildet. Dimethylstyrole lassen sich, gemäss DE-AS 16971722, durch Umsetzung von Xylolen mit Aethylen und Sauerstoff in Gegenwart einer Palladiumverbindung einer niederen aliphatischen Carbonsäure herstellen. Die Dimethylstyrole werden dabei mit einer Salzaktivität von 60-90 % gebildet.

Andererseits ist bekannt, dass die Umsetzung von Benzoylchlorid mit Acrylsäuremethylester in Gegenwart stöchiometrischer Mengen eines Nickel(O)-Katalysators bei Nachbehandlung des Reaktionsgemisches mit Jod in Methanol zur Bildung von trans-3-Benzoylacrylsäuremethylester führt. Als Nebenprodukt fällt dabei Zimtsauremethylester an. Durch Umsetzung eines Komplexes aus Benzoylpalladiumchlorid und Triphenylphosphin mit Acrylsäuremethylester bei 70 bis 85 °C in Gegenwart von Triäthylamin erhält man Zimtsäuremethylester als Hauptprodukt und Benzoylacrylsäuremethylester als Nebenprodukt. Werden das Palladium und das Triphenylphosphin nur in katalytischen Mengen eingesetzt, so verschiebt sich das Reaktiongleichgewicht zu Gunsten der Bildung des Benzoylacrylsäuremethylesters (Gewichtsverhältnis von Benzoylacrylsäuremethylester : Zimtsäuremethylester = ca. 8,3 : 1) [vgl. Transition Met. Chem. *2*, 270 (1977) und *4*, 298 (1979)]. Schliesslich ist aus Synthesis, 777 (1977) bekannt, dass die Umsetzung von aromatischen Säurehalogeniden mit 1-Alkinen unter Pd-Katalyse ohne Decarbonylierung zu Alkinylketonen führt.

Vinylisch substituierte Stilbene sind aus der DE-OS 2602750 bzw. US Patentschrift 4108887 bekannt. Sie werden nach an sich bekannten Methoden aus sehr schwer zugänglichen Ausgangsstoffen [vgl. Chem. Ber. *91*, 1278 (1958)] hergestellt.

Es wurde gefunden, dass man Verbindungen der Formel Ia oder Ib

$$Z{-}CH = CH{-}Z_1 \text{ (Ia) oder } CH_2 = CH{-}Z_2{-}CH = CH_2 \qquad \text{(Ib)}$$

worin

Z unsubstituiertes oder substituiertes Phenyl oder Naphthyl darstellt,

$Z_1$ Wasserstoff ist oder dieselbe Bedeutung wie Z hat und

$Z_2$ unsubstituiertes oder substituiertes Phenylen, Naphthylen oder p-Biphenylen oder einen unsubstituierten oder substituierten Stilbenrest bedeutet, dadurch herstellen kann, dass man bei einem Druck von 0,1 bis 20 bar Aethylen in Gegenwart einer Base und unter Zusatz von Palladium-Metall oder von Palladiumverbindungen, die unter den Reaktionsbedingungen phosphorfreie labile Palladium (O) Verbindungen bilden, als Katalysator, mit einer Verbindung der Formel II oder III

$$Z{-}COX \text{ (II) oder } XOC{-}Z_2{-}COX \qquad \text{(III)},$$

umsetzt, wobei Z und $Z_2$ die unter Formel Ia bzw. Ib angegebenen Bedeutungen haben und X Chlor, Brom oder Jod darstellt.

Nach dem erfindungsgemässen Verfahren lassen sich die Verbindungen der Formel I auf einfache, wirtschaftliche Weise unter milden Reaktionsbedingungen und unter Verwendung von leicht zugänglichen Ausgangsprodukten herstellen. Dabei verläuft die Reaktion überraschenderweise selektiv unter Decarbonylierung der Säurehalogenide der Formel II oder III.

Die in Gruppen Z, $Z_1$ oder $Z_2$ vorkommenden Substituenten sind solche, die unter den Reaktionsbedingungen inert sind. Die genannten Gruppen Z, $Z_1$ oder $Z_2$ können ein- oder mehrfach substituiert sein.

Als Substituenten an Gruppen Z, $Z_1$ und $Z_2$ kommen z. B. in Betracht : Halogenatome, Formyl, $-CH(OCH_3)_2$, $-CH(OC_2H_5)_2$,

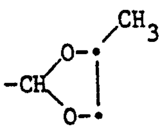 , 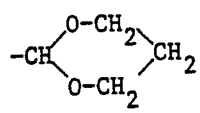 , 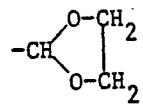 ,

$-C(X)_1=C(X_2)(X_3)$, worin $X_1$ Wasserstoff, $C_{1-4}$-Alkyl, —CN oder —COOR, $X_2$ Wasserstoff, $C_{1-4}$-Alkyl, $-(CH_2)_m-COOR$ oder $-(CH_2)_m-CN$ mit m = 1 bis 4, $X_3$ Phenyl, —CN, —COOR oder $-CON(R)_2$ und die R unabhängig voneinander $C_{1-12}$-Alkyl oder Phenyl darstellen, wobei mindestens eines von $X_1$ und $X_2$ Wasserstoff sein muss ;

$C_{1-10}$-Alkyl-, $C_{1-16}$-Alkoxy-, Phenoxy-, Di($C_{1-10}$-alkyl)amino-, Nitro-, Cyano- ; $-CH_2Cl-$, Trifluormethyl-, Benzyl-, $C_{1-4}$-Alkyl-sulfonyl-, $-CO-C_{1-10}$-Alkyl, —CO-Phenyl-, $-O-\overset{\|}{\underset{O}{C}}-C_{1-10}$-Alkyl-, $-COO-C_{1-10}$-Alkyl-, COO-Phenyl-, Phenyl- oder Naphthylgruppen, die ihrerseits durch Halogenatome, $C_{1-10}$-Alkyl-, $C_{1-10}$-Alkoxy-, Di-($C_{1-10}$-alkyl)amino-, Nitro-, Cyano-, Trifluormethyl-, $-CO-C_{1-10}$-Alkyl-, —CO-Phenyl-, $-COO-C_{1-10}$-Alkyl- oder —COO-Phenylgruppen substituiert sein können. Phenyl- und Naphthylsubstituenten an Gruppen Z, $Z_1$ oder $Z_2$ sind bevorzugt monosubstituiert oder unsubstituiert. Alkyl- und Alkoxygruppen in den oben erwähnten Substituenten können geradkettig oder verzweigt sein, wobei Alkyl- und Alkoxysubstituenten an Gruppen Z, $Z_1$ oder $Z_2$ bevorzugt 1 bis 8 und insbesondere 1 bis 4 C-Atome aufweisen. Halogensubstituenten sind z. B. Fluor, Chlor oder Brom. Als Beispiele definitionsgemässer Substituenten an Gruppen Z, $Z_1$ oder $Z_2$ seien erwähnt : die Methyl-, Aethyl-, n-Propyl-, Isopropyl-, n-, sek.- und tert.-Butyl-, n-Pentyl-, 2-Pentyl-, n-Hexyl-, n-Heptyl, n-Octyl- und n-Decylgruppe ; die Methoxy-, Aethoxy-, n-Propoxy-, n-Butoxy-, n-Hexyloxy- und n-Decyloxygruppe ; die N,N-Dimethylamino-, N,N-Diäthylamino-, N,N-Di-n-propylamino-, N,N-Di-n-butylamino-, N,N-Di-n-hexylamino-, N,N-Di-n-octylamino-, N-Methyl-N-äthylamino-, N-Methyl-N-n-propylamino-, N-Aethyl-N-n-hexylamino- und N-Aethyl-N-n-butylaminogruppe ; die Methyl- und Aethylsulfonylgruppe ; die Acetyl-,Propionyl-, Butyryl-, Valeroyl- und Octanoylgruppe ; die Carbonsäuremethyl-, äthyl-, -n-propyl-, -isopropyl-, -n-butyl-, -n-pentyl-, -n-hexyl-, -n-heptyl- und -n-decylestergruppe ; die Gruppen —CH=CH-Phenyl, $-CH=C(COOCH_3)CH_2COOCH_3$, $-CH=C(CH_3)COOC_2H_5$, $-C(COOCH_3)=CHCOOCH_3$, $-CH=C(CN)CH_2CH_2CN$, —CH=CHCOOR und —CH=CHCN, wobei R die oben angegebene Bedeutung hat.

X in den Formeln II und III stellt vorzugsweise Brom und insbesondere Chlor dar. Alkylgruppen R können geradkettig oder verzweigt sein. Bevorzugt sind geradkettige Alkylgruppen R mit 1-4 und vor allem 1 oder 2 C-Atomen.

Bevorzugt herstellbare Verbindungen der Formel Ia sind die Verbindungen der Formeln

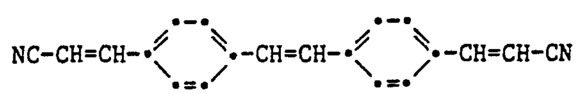

und

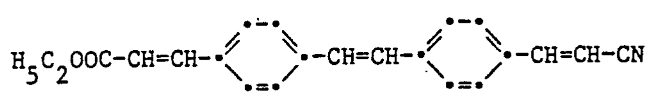

sowie die neue Verbindung der Formel

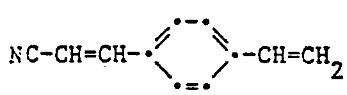

Als Verbindungen der Formeln II und III kommen insbesondere in Betracht :

1. Verbindungen der Formel IIa

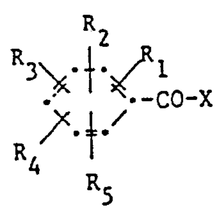 (IIa)

worin X Chlor oder Brom, $R_1$ Wasserstoff, Cl, Br, F, J, Formyl, $-CH(OCH_3)_2$, $-CH(OC_2H_5)_2$,

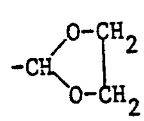 ,

—CH=CHCN, —CH=CH—COO—$C_{1-4}$-Alkyl, $C_{1-4}$-Alkyl, $C_{1-4}$-Alkoxy, Phenoxy, Di($C_{1-2}$-alkyl)amino, —$NO_2$, —CN, —$CF_3$, $C_{1-4}$-Alkylsulfonyl, Benzyl, —CO—$C_{1-4}$-Alkyl, —CO-Phenyl, —OCO—$C_{1-4}$-Alkyl, —COO—$C_{1-4}$-Alkyl, —COO-Phenyl, Phenyl, Chlorphenyl, Bromphenyl, Methylphenyl, Methoxyphenyl, 1- oder 2-Naphthyl, $R_2$ und $R_3$ unabhängig voneinander Wasserstoff, Cl, Br, F, —$NO_2$, $C_{1-4}$-Alkyl oder $C_{1-4}$-Alkoxy, besonders Methyl oder Methoxy, $R_4$ und $R_5$ Wasserstoff oder, wenn $R_1$, $R_2$ und $R_3$ je Chlor, Brom, Fluor oder Methyl sind, ebenfalls je Chlor, Brom, Fluor oder Methyl bedeuten.

Bevorzugt sind Verbindungen der Formel IIa, worin X Chlor, $R_1$ Wasserstoff, Cl, Br, F, J, —CH=CHCN, —CH=$CHCOOCH_3$ —CH=$CHCOOC_2H_5$, $C_{1-4}$-Alkyl, besonders Methyl oder Aethyl, Methoxy, N,N-Dimethyl-amino, —$NO_2$, —CN, Formyl, Methylsulfonyl oder Phenyl, $R_2$ Wasserstoff, Cl, Br, Methyl, Aethyl, Methoxy oder Nitro, $R_3$ Wasserstoff, Cl, Br, Methyl, Aethyl oder Methoxy und $R_4$ und $R_5$ je Wasserstoff bedeuten und besonders solche worin X Chlor, $R_1$ —CH=CHCN oder —CH=CH—$COOC_2H_5$ und $R_2$ bis $R_5$ je Wasserstoff bedeuten.

2. Verbindungen der Formel IIb

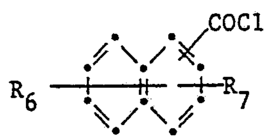
(IIb)

worin die Gruppe —COCl in 1- oder 2-Stellung ist, $R_6$ und $R_7$ an denselben Ring oder an verschiedene Ringe gebunden sein können, $R_6$ Wasserstoff, Cl, Br, F, Methyl, Aethyl, Methoxy, Aethoxy, —CHO, —$COCH_3$, —$SO_2CH_3$, —CN, —$NO_2$ oder —$CH(OCH_3)_2$ und $R_7$ Wasserstoff, Cl, Br, F, Methyl, Methoxy oder —$NO_2$ bedeuten. Als Verbindungen der Formel IIb werden solche bevorzugt, worin $R_6$ Methyl und insbesondere Wasserstoff und $R_7$ Wasserstoff darstellen.

3. Verbindungen der Formel IIIa

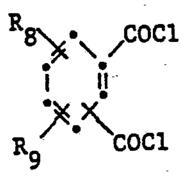
(IIIa)

worin $R_8$ Wasserstoff, —CO-Phenyl, Cl, Br, F, —CN, —CHO, —$NO_2$ oder Methyl und $R_9$ Wasserstoff, Cl, Br, F oder Methyl bedeuten. Als Verbindungen der Formel IIIa werden Iso- und Terephthalsäuredichlorid, die gegebenenfalls durch eine Methyl- oder $NO_2$-Gruppe substituiert sind und vor allem das unsubstituierte Iso- oder Terephthalsäuredichlorid, bevorzugt.

4. Verbindungen der Formel IIIb

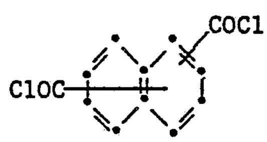
(IIIb)

wobei die —COCl-Gruppen an denselben Ring oder an verschiedene Ringe gebunden sein können. Als Verbindungen der Formel IIIb werden 1,4- und 2,6-Naphthalindicarbonsäuredichlorid bevorzugt.

5. Die Verbindungen der Formeln IIIc und IIId

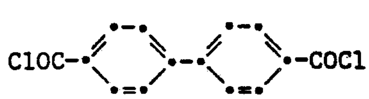
und
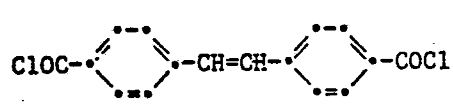

(IIIc) (IIId)

Besonders bevorzugt als Säurehalogenide sind die Verbindungen der Formeln IIIc und IIId sowie Verbindungen der Formel IIa, worin X Chlor, $R_1$ Wasserstoff, Formyl, Methyl, Methoxy, Cl, Br oder Nitro, $R_2$ Wasserstoff, Methyl, Methoxy, Cl oder Br, $R_3$ Wasserstoff oder Methoxy und $R_4$ und $R_5$ Wasserstoff bedeuten oder worin X Chlor darstellt, $R_1$ —CH=CHCN, —CH=$CHCOOCH_3$ oder —CH=$CHCOOC_2H_5$ ist und $R_2$ bis $R_5$ Wasserstoff bedeuten.

Beim erfindungsgemässen Verfahren können bei der Umsetzung mit Säurehalogeniden der Formel II je nach Reaktionsbedingungen Gemische von Styrolderivaten ($Z_1$ = H) und Stilbenen entstehen. Die Reaktion lässt sich hauptsächlich durch Variation des angewendeten Druckes steuern. Stilbene werden

vornehmlich bei einem Druck zwischen 0,1 und 1 bar vorzugsweise bei 1 bar (Normaldruck) gebildet, während bei höherem Druck zweckmässig einem Druck zwischen 5 und 15 bar, vorzugsweise bei 10 bar, zur Hauptsache Styrolderivate entstehen. Die Umsetzung mit den Säurehalogeniden der Formel III zu Verbindungen der Formel Ib wird vorzugsweise unter Druck, besonders einem Druck zwischen 5 und 15 bar vorzugsweise 10 bar, vorgenommen.

Die Katalysatoren sowie die Verbindungen der Formeln II und III sind bekannt oder können nach an sich bekannten Methoden hergestellt werden. Bezüglich der Herstellung von Verbindungen der Formeln II und III vgl. z. B. « Organikum », 387-388, VEB Deutscher Verlag der Wissenschaften, Berlin 1964.

Als definitionsgemässe Palladiumverbindungen können — neben Palladium-Metall — z. B. Verbindungen der Formel IV

$$M^y [PdL_n]^x \qquad (IV)$$

eingesetzt werden, worin n eine ganze Zahl von 2 bis 4, x $2^\oplus$ bis $2^\ominus$, y = − (x), M bei x ungleich O, ein Gegenion und die L gleiche oder verschiedene phosphorfreie Liganden darstellen, wie z. B. Cl, Br, J, —CN, —$NO_3$, $C_{1-12}$-Alkyl-COO, $CH_3COCHCOCH_3$, $NH_3$, 2,2'-Bipyridyl, o-Phenanthrolin, $OS(CH_3)_2$ oder —NC-Phenyl. Geeignete Verbindungen der Formel IV sind beispielsweise $PdCl_2$, $PdBr_2$, $Pd(CN)_2$, $Pd(NO_3)_2$, $Pd(O_2C\text{-}C_{1-12}\text{-Alkyl})_2$, besonders $Pd(OOCCH_3)_2$, $Pd(CH_3COCHCOCH_3)_2$, $[Pd(NH_3)_4]Cl_2$, $[PdCl_4]Na_2 \cdot Pd(OOCCH_3)_2$ (2,2'-Bipyridyl), $Pd(OOCCH_3)_2$-(0-Phenanthrolin), $PdCl_2[OS(CH_3)_2]_2$ und $PdCl_2$(NC-Phenyl)$_2$.

Ausser den oben genannten Verbindungen können auch Palladiumverbindungen anderer Oxidationsstufen eingesetzt werden, z. B. Bis-(Dibenzylidenaceton) Palladium (0) und Bis-(Isonitril) Palladium (0)-Verbindungen. Als Beispiele solcher Isonitrile seien genannt : Bis-(Cyclohexylisonitril) Palladium (0), Bis-(Isopropylisonitril)-Palladium (0), Bis-(tert.-Butylisonitril) Palladium (0), Bis-(p-Tolylisonitril) Palladium (0), Bis-(Phenylisonitril) Palladium (0) und Bis-(p-Methoxyphenyl-isonitril) Palladium (0). Davon sind Bis-(Dibenzylidenaceton) Palladium (0), Bis-(Cyclohexylisonitril) Palladium (0) und Bis-(Isopropylisonitril) Palladium (0) bevorzugt.

Als Katalysatoren verwendet man bevorzugt $PdCl_2$, $PdBr_2$, $Pd(OOCCH_3)_2$, $Pd(CH_3COCHCOCH_3)_2$, $Pd(OOCCH_3)_2$ (2,2'-Bipyridyl), $PdCl_2$(NC-Phenyl)$_2$, Bis-(Dibenzylidenaceton) Palladium (0) und Bis-(Cyclohexylisonitril)-Palladium (0). Ganz besonders bevorzugt sind $PdCl_2$, Palladiumacetat und Bis-(Dibenzylidenaceton) Palladium (0). Die Katalysatoren werden im allgemeinen in einer Menge von 0,000 1 bis 20 Mol.%, bevorzugt 0,001 bis 3 Mol.%, bezogen auf die Verbindung der Formel II bzw. III, eingesetzt.

Als Basen können im erfindungsgemässen Verfahren sowohl anorganische als auch organische Verbindungen, die im Reaktionsmedium ausreichend löslich sind, verwendet werden. Geeignete Basen sind beispielsweise Verbindungen der Formeln V bis VII

$(Z')^{n\oplus}\ (^{\ominus}OOCQ')_n$ , 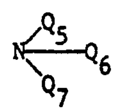 oder 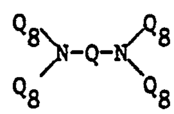

(V) (VI) (VII)

sowie cyclische tertiäre Amine, z. B. N-Methyl- oder N-Aethylpiperidin, 1,2,2,6,6-Pentamethylpiperidin, 4-Oxo-1,2,2,6,6-pentamethylpiperidin, 1,4-Diazabicyclo [2.2.2] octan (DABCO), N-Alkylmorpholine und N-Alkylpyrrolidine, wie N-Methyl- und N-Aethylmorpholine, N-Methyl- und N-Aethylpyrrolidin, oder N,N'-Dialkylpiperazine, wie N,N'-Dimethylpiperazin.

In den obigen Formeln bedeuten :

n die Zahl 1 oder 2,

Q' Phenyl oder $C_{1-17}$-Alkyl,

Z' ein Alkalimetallkation, ein Erdalkalimetallkation oder

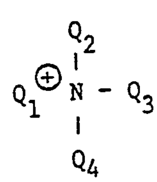 ,

Q geradkettiges oder verzweigtes Alkylen mit 2-6 C-Atomen,

Q' Wasserstoff, $C_{1-12}$-Alkyl, Cyclopentyl, Cyclohexyl, Benzyl oder Phenyl,

$Q_2$, $Q_3$ und $Q_4$ gleiches oder verschiedenes $C_{1-12}$-Alkyl,

$Q_5$ $C_{1-12}$-Alkyl, Cyclopentyl, Cyclohexyl, Phenyl oder Benzyl, das auch substituiert sein kann, beispielsweise durch ein Halogenatom, wie Chlor oder Brom, eine Alkyl- oder Alkoxygruppe mit je 1-4 und besonders 1 oder 2 C-Atomen,

$Q_6$ und $Q_7$ gleiches oder verschiedenes $C_{1-12}$-Alkyl und
$Q_8$ Methyl oder Aethyl.

Z' in der Bedeutung eines Alkalimetallkations ist besonders das Natrium- und vor allem das Lithiumkation. Alkylgruppen Q' und $Q_1$ bis $Q_7$ können geradkettig oder verweigt sein. Stellen $Q_5$ bis $Q_7$ Alkylgruppen dar, so weisen diese mit Vorteil zusammen mindestens 9 C-Atome auf, während Alkylgruppen $Q_1$ bis $Q_4$ bevorzugt je 1-4 C-Atome aufweisen. Beispiele von Verbindungen der Formel V bis VII sind : das Lithiumacetat, -butyrat und -stearat, das Barium- und Calciumacetat, das Kalium-, Calcium- und Natriumstearat, das Lithium- und Natriumbenzoat, sowie die entsprechenden Trimethyl-, Tetramethyl-, Tetraäthyl- und Tetra-n-butylammoniumsalze ; Triäthylamin, Tri-n-butylamin, Tri-(2-äthylhexylamin), Tri-n-octylamin und Tri-n-dodecylamin ; N-Benzyldialkylamine, wie N-Benzyldimethylamin- N-Benzyldiäthylamin, N-(4-Chlorbenzyl)-dimethylamin und N-(3-Methyl- oder 3-Methoxybenzyl)-dimethylamin ; N,N,N',N'-Tetramethyl- und N,N,N',N'-Tetraäthyl-äthylendiamin, N,N,N',N'-Tetramethyl-1,3-diaminopropan und N,N,N',N'-Tetramethyl-1,6-diaminohexan.

Bevorzugt verwendet man als Basen tertiäre Amine der vorerwähnten Art, vor allem N-Aethylmorpholin oder Verbindungen der Formel VI, worin $Q_5$ 4-Chlorbenzyl, 4-Methylbenzyl oder 4-Methoxybenzyl und insbesondere Benzyl und $Q_6$ und $Q_7$ je Alkyl mit 1-4 C-Atomen, vor allem 1 oder 2 C-Atomen, darstellen, oder worin $Q_5$, $Q_6$ und $Q_7$ je Alkyl mit 3-12 C-Atomen darstellen. Besonders bevorzugt sind N-Benzyldimethylamin, N-Aethylmorpholin und Tri-n-butylamin.

Die Reaktionstemperaturen für die erfindungsgemässe Umsetzung liegen zwecksmässig zwischen 0 und 200 °C, vorzugsweise zwischen 80 und 150 °C. Sind die Verbindungen der Formeln II oder III flüssig, so kann die Umsetzung ohne Zusatz eines Lösungsmittels durchgeführt werden. Bevorzugt wird die Reaktion jedoch in einem gegenüber den Reaktionspartnern inerten organischen Lösungsmittel durchgeführt. Geeignete inerte organische Lösungsmittel sind je nach Reaktionskomponenten z. B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol ; aromatische, aliphatische und cyclische Aether, wie Anisol, Diäthyläther, Di-isopropyläther, Tetrahydrofuran und Dioxan ; Nitrile, besonders Benzonitril und Alkylnitrile mit 2 bis 5 C-Atomen, wie Propionitril und Butyronitril ; 3-Methoxypropionitril und 3-Aethoxypropionitril ; Dialkylsulfoxide, wie Dimethyl- und Diäthylsulfoxid ; N,N-Dialkylamide von aliphatischen Monocarbonsäuren mit 1-3 C-Atomen im Säureteil, wie N,N-Dimethylformamid und N,N-Dimethylacetamid ; Alkohole mit bis zu 8 C-Atomen, wie Aethanol, n-Propanol und tert.-Butanol ; aliphatische und cycloaliphatische Ketone, wie Aceton, Diäthylketon, Methylisopropylketon, Cyclopentanon und Cyclohexanon ; Ester, wie Ester der Kohlensäure, z. B. Diäthylcarbonat ; Nitromethan ; Alkyl- oder Alkoxyalkylester von aliphatischen Monocarbonsäuren mit insgesamt 2-8 C-Atomen, wie Essigsäuremethyl-, -äthyl-, -n-butyl- und -isobutylester, Buttersäureäthyl- und -n-butylester sowie 1-Acetoxy-2-äthoxyäthan und 1-Acetoxy-2-methoxyäthan. Bevorzugte Lösungsmittel sind Ketone, Ester, aromatische und cyclische Aether und aromatische Kohlenwasserstoffe der oben erwähnten Art. Für die Umsetzung in Gegenwart anorganischer Basen eignen sich vor allem polare Lösungsmittel, wie Ketone und Ester. Ganz besonders bevorzugt wird die Umsetzung in gegenwart eines aromatischen Aethers oder Kohlenwasserstoffs, vor allem Anisol, Xylolen oder Toluol, vorgenommen.

Die erfindungsgemäss herstellbaren Verbindungen und deren Anwendungen sind zum grossen Teil bekannt. Sie können beispielsweise direkt als optische Aufheller oder aber als Zwischenprodukte für optische Aufheller oder Scintillatoren eingesetzt werden. Derartige optische Aufheller bzw. Scintillatoren sind z. B. in der U.S. Patentschrift 4 108 887 und der britischen Patentschrift 2 015 021 beschrieben. Die erfindungsgemäss hergestellten Verbindungen können ferner in an sich bekannter Weise, gegebenenfalls unter Einführung von geeigneten funktionellen Gruppen, wie Aminogruppen, und/oder durch Sulfonierung der aromatischen Reste Z, $Z_1$ und $Z_2$ in Farbstoffe oder optische Aufheller übergeführt werden [vgl. z. B. Encyclopedia of Chemical Technology, 2. Auflage Bd. 19, S. 1 bis 16]. Stilbene und Stilbenderivate finden auch Anwendung als Klebstoffadditive, Insektizide oder Lichtstabilisatoren ; vgl. z. B. Chemical Abstracts *78*, 39352 ; *84*, 137386 und *85*, 22416. Styrole und Styrolderivate eignen sich auch zur Herstellung von Homo- oder Copolymeren.

In den folgenden Beispielen wurden die Umsetzungen, sofern nichts anderes angegeben ist, bei Normaldruck (ca. 1 bar) durchgeführt.

## Beispiel 1

Zu 100 ml Toluol werden 5,76 ml (50 mMol) Benzoylchlorid, 7,53 ml (50 mMol) N-Benzyldimethylamin und 0,112 2 g (0,5 mMol) Palladiumacetat gegeben. Aethylen wird durch das Reaktionsgemisch geleitet. Anschliessend wird 8 Stunden bei 100 °C gerührt. Gemäss gaschromatographischer Analyse enthält das Reaktionsgemisch 37 % trans-Stilben und 18 % Styrol.

## Beispiele 2-4

Beispiel 1 wird wiederholt unter Verwendung anderer Lösungsmittel :

| Bsp. | Lösungsmittel | % trans-Stilben | % Styrol |
|---|---|---|---|
| 2 | 1,4-Dioxan | 15 | 4 |
| 3 | Cyclohexanon | 13 | 0 |
| 4 | Chlorbenzol | 24 | nicht bestimmt. |

Beispiele 5-13

Beispiel 1 wird wiederholt, unter Abänderung einiger Verfahrensparameter wie unten angegeben :

| Bsp. | | % trans-Stilben | % Styrol |
|---|---|---|---|
| 5 | Rühren bei 80°C | 14 | 24 |
| 6 | 100 ml p-Xylol, bei 120°C gerührt | 60 | 30 |
| 7 | wie Bsp. 6 und mit 5,75 g (50 mMol) N-Aethylmorpholin | 54 | 32 |
| 8 | wie Bsp. 6 und mit 9,27 g (50 mMol) Tri-n-butylamin | 35 | 28 |
| 9 | wie Bsp. 6 mit 0,190 g (0,5 mMol) Diacetatobipyridyl-palladium(II) | 51 | 24 |
| 10 | wie Bsp. 6, bei einem Druck von 0,15 bar, Reaktionszeit 2 Stunden | 55 | 34 |
| 11 | wie Bsp. 6, bei einem Druck von 1,28 bar, Reaktionszeit 2 Stunden | 42 | 38 |
| 12 | wie Bsp. 6 mit 0,0112 g (0,05 mMol) Palladiumacetat | 26 | nicht bestimmt |
| 13 | wie Bsp. 6, Rühren bei 130°C | 60 | nicht bestimmt |

Beispiel 14

Zu 400 ml p-Xylol werden 34,1 g (0,2 Mol) 4-Chlorbenzoylchlorid, 27,04 g (0,2 Mol) N-Benzyldimethylamin und 0,448 g (2 mMol) Palladiumacetat zugegeben. Aethylen wird durch das Reaktionsgemisch geleitet und das Gemisch wird 6 Stunden bei 120 °C gerührt. Dann wird das Gemisch filtriert, und das Filtrat wird eingedampft. Der Rückstand wird am Filter mit 200 ml Methanol gewaschen, um das entstandene Aminsalz zu entfernen. Anschliessend wird aus 400 ml Cyclohexan umkristallisiert. Man erhält 13,7 g (56 % d. Th.) 4,4'-Dichlorstilben (trans) in Form weisser Kristalle ; Fp. 176,2 °C.

Beispiel 15

Beispiel 14 wird wiederholt unter Verwendung von 30,92 g (0,2 Mol) p-Toluylchlorid. Das Gemisch wird 8 Stunden bei 120 °C gerührt. Nach dem Aufarbeiten wie in Beispiel 14 beschrieben und dem Umkristallisieren erhält man 9,3 g (45 % d. Th.) 4,4'-Dimethylstilben (trans) in Form weisser glänzender Kristalle ; Fp. 184,5 °C.

Beispiel 16

Beispiel 14 wird wiederholt unter Verwendung von 46,13 g (0,2 Mol) 3,4,5-Trimethoxybenzoylchlorid.

Das Gemisch wird 4,5 Stunden bei 120 °C gerührt. Nach der Aufarbeitung wie in Beispiel 14 beschrieben und zweimaligem Umkristallisieren (einmal aus 200 ml Cyclohexan und 500 ml Toluol und einmal aus 900 ml Aceton) erhält man 10,7 g (30 % d. Th.) 3,3',4,4',5,5'-Hexamethoxystilben (trans) in Form gelber Kristalle ; Fp. 220,4 °C.

Beispiel 17

Beispiel 14 wird wiederholt unter Verwendung von 33,7 g (0,2 Mol) 3,4-Dimethylbenzoylchlorid. Das Gemisch wird 6,5 Stunden bei 120 °C gerührt. Nach dem Aufarbeiten wie in Beispiel 14 beschrieben und dem Umkristallisieren aus 250 ml n-Hexan erhält man 10,2 g (43 % d. Th.) 3,3',4,4'-Tetramethylstilben (trans) als hellgelbe Kristalle ; Fp. 137,6 °C.

Beispiel 18

Beispiel 14 wird wiederholt unter Verwendung von 34,1 g (0,2 Mol) 3-Methoxybenzoylchlorid. Das Gemisch wird 7 Stunden bei 120 °C gerührt. Nach dem Aufarbeiten wie in Beispiel 14 beschrieben und dem Umkristallisieren aus 200 ml n-Hexan erhält man 10,0 g (42 % d. Th.) 3,3'-Dimethoxystilben (trans) in Form hellgelber Kristalle ; Fp. 98,4 °C.

Beispiel 19

Beispiel 14 wird wiederholt unter Verwendung von 41,89 g (0,2 Mol) 3,4-Dichlorbenzoylchlorid. Das Gemisch wird 6 1/4 Stunden bei 120 °C gerührt. Nach dem Aufarbeiten wie in Beispiel 14 beschrieben und dem Umkristallisieren aus einem Gemisch von 300 ml Cyclohexan und 250 ml Tetrachlorkohlenstoff erhält man 10,1 g (32 % d. Th.) 3,3',4,4'-Tetrachlorstilben (trans) in Form hellbrauner Kristalle ; Fp. 187,7 °C.

Beispiel 20

Beispiel 14 wird wiederholt unter Verwendung von 37,11 g (0,2 Mol) 4-Nitrobenzoylchlorid und 23,04 g (0,2 Mol) N-Aethylmorpholin. Das Gemisch wird 6 Stunden bei 120 °C gerührt. Nach dem Aufarbeiten gemäss Beispiel 14 und zweimaligem Umkristallisieren aus je 50 ml N,N-Dimethylformamid erhält man 8,65 g (32 % d. Th.) 4,4'-Dinitrostilben (trans) in Form gelber Nadeln ; Fp. > 300 °C.

Beispiel 21

Beispiel 20 wird wiederholt unter Verwendung von 39,9 g (0,2 Mol) 2-Methyl-3-nitrobenzoylchlorid anstelle von 4-Chlorbenzoylchlorid. Das Gemisch wird auf 5 °C gekühlt, das Rohprodukt wird abfiltriert und mit 200 ml 2N HCl gewaschen. Anschliessend wird einmal aus 200 ml N,N-Dimethylformamid und einmal aus 100 ml N,N-Dimethylformamid umkristallisiert. Man erhält 13,1 g (44 % d. Th.) 2,2'-Dimethyl-3,3'-dinitrostilben (trans) in Form hellgelber Kristalle ; Fp. 246,0 °C.

Beispiel 22

Zu 100 ml p-Xylol werden 8,43 g (50 mMol) 4-Formylbenzoylchlorid, 11,91 ml (50 mMol) Tri-n-butylamin und 0,112 2 g (0,5 mMol) Palladiumacetat gegeben. Aethylen wird durch das Gemisch geleitet, und das Gemisch wird 3 Stunden bei 120 °C gerührt. Dann wird das Gemisch mit 100 ml 2N HCl ausgeschüttelt und mit 5 g Magnesiumsulfat getrocknet. Nach dem Eindampfen wird der braune Rückstand auf Kieselgel chromatographiert (Laufmittel : Dichlormethan). Es werden zwei Produkte erhalten. Das erste wird nach dem Abdampfen des Dichlormethans in einem Kugelrohr bei 55 °C/0,002 bar destilliert. Man erhält 0,32 g (5 % d. Th.) 4-Formylstyrol. Das zweite Reaktionsprodukt wird nach dem Entfernen des Dichlormethans aus Benzol/Cyclohexan umkristallisiert. Man erhält 0,57 g (10 % d. Th.) 4,4'-Diformylstilben in Form gelber Blättchen ; Fp. 168,9 °C.

Gemäss Beispielen 14-21 wurden stets kleinere Anteile der entsprechenden Styrole gebildet, die aber nicht isoliert wurden.

Beispiel 23

Zu 25 ml Toluol werden in einer Druckapparatur aus Glas 1,44 ml (12,5 mMol) Benzoylchlorid, 1,88 ml (12,5 mMol) N-Benzyldimethylamin und 0,028 1 g (0,125 mMol) Palladiumacetat gegeben. Die Apparatur wird mit Aethylen gespült, um die Luft zu entfernen. Dann wird bei 10 bar Aethylen aufgepresst und 4 Stunden bei 120 °C gerührt. Es werden 55 % Styrol und 9 % trans-Stilben gebildet.

## Beispiele 24-38

Analog der in Beispiel 23 beschriebenen Arbeitsweise werden unter Abänderung einiger Verfahrensparameter wie unten angegeben die folgenden Anteile an Styrol bzw. trans-Stilben erhalten :

| Bsp. | | % Styrol | % trans-Stilben |
|---|---|---|---|
| 24 | bei 100°C gerührt | 58 | 7 |
| 25 | bei 140°C gerührt | 65 | 8 |
| 26 | bei 80°C gerührt | 27 | 2 |
| 27 | 1,44 g (12,5 mMol) N-Aethylmorpholin anstelle von N-Benzyldimethylamin | 2 | 0 |
| 28 | 2,32 g (12,5 mMol) Tri-n-butylamin anstelle von N-Benzyldimethylamin | 27 | 2 |
| 29 | Aethylendruck 15 bar | 37 | 0 |
| 30 | Aethylendruck 5 bar | 35 | 4 |
| 31 | 25 ml Dioxan anstelle von Toluol | 41 | 2 |
| 32 | 25 ml Propionitril anstelle von Toluol | 32 | 2 |
| 33 | 25 ml Essigsäureäthylester anstelle von Toluol | 42 | 2 |
| 34 | 0,0028 g (0,0125 mMol) Palladiumacetat | 8 | 0 |
| 35 | 2 Stunden gerührt | 40 | nicht bestimmt |
| 36 | 6 Stunden gerührt | 57 | 2 |
| 37 | 0,0380 g (0,125 mMol) Bis-(acetylacetonato)Palladium(O) | 48 | 2 |
| 38 | 0,0480 g (0,125 mMol) Dichlor-bis-(benzonitril)Palladium(II) | 48 | nicht bestimmt. |

## Beispiel 39

Zu 100 ml Toluol werden 7,73 g (50 mMol) p-Toluylchlorid, 6,76 g (50 mMol) N-Benzyldimethylamin und 0,112 g (0,5 mMol) Palladiumacetat gegeben. Das Gemisch wird 4 Stunden bei 140 °C und einem Aethylendruck von 10 bar gerührt. Das Reaktionsgemisch wird filtriert, das Filtrat wird mit 100 ml 2N HCl, 100 ml 2N NaOH und dann mit 30 ml konz. wässrigem Ammoniak und 50 ml Wasser ausgeschüttelt. Nach dem Trocken mit Magnesiumsulfat wird das Gemisch eingedampft und auf Kieselgel chromatographiert. Nach dem Eindampfen erhält man 2,1 g (36 % d. Th.) 4-Methylstyrol als farblose Flüssigkeit. Zur Identifizierung wird Brom an die Doppelbindung addiert :

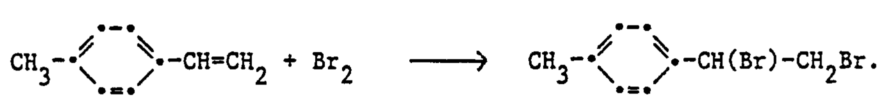

Man erhält das Bromderivat in Form weisser Kristalle ; Fp. 44,7 °C.

Beispiel 40

Beispiel 39 wird wiederholt unter Verwendung von 8,75 g (50 mMol) 4-Chlorbenzoylchlorid anstelle von p-Toluylchlorid. Nach dem Aufarbeiten wie in Beispiel 39 beschrieben erhält man 3,8 g (55 % d. Th.) 4-Chlorstyrol als farblose Flüssigkeit, deren entsprechendes Bromderivat in Form von weissen Kristallen vom Fp. 46,0 °C ausfällt.

Beispiel 41

Beispiel 39 wird wiederholt unter Verwendung von 8,53 g (50 mMol) 3-Methoxybenzoylchlorid. Nach dem Aufarbeiten gemäss Beispiel 39 erhält man 3,2 g (48 % d. Th.) 3-Methoxystyrol als farblose Flüssigkeit, deren entsprechendes Bromderivat in Form von weissen Kristallen vom Fp. 63,3 °C ausfällt.

Beispiel 42

Beispiel 39 wird wiederholt unter Verwendung von 11,53 g (50 mMol) 3,4,5-Trimethoxybenzoylchlorid. Nach dem Aufarbeiten gemäss Beispiel 39 erhält man 4,1 g (36 % d. Th.) 3,4,5-Trimethoxystyrol als gelbe Flüssigkeit. Das 3,4,5-Trimethoxystyrol wird in das entsprèchende Bromderivat übergeführt (hellgelbe Nadeln vom F. 104,1 °C).

Beispiel 43

Beispiel 39 wird wiederholt unter Verwendung von 10,47 g (50 mMol) 3,4-Dichlorbenzoylchlorid. Nach dem Aufarbeiten gemäss Beispiel 39 erhält man 1,6 g (18 % d. Th.) 3,4-Dichlorstyrol als gelbe Flüssigkeit. Durch Ueberführen in das entsprechende Bromderivat erhält man weisse Kristalle vom Fp. 49,3 °C.

Beispiel 44

Analog der in Beispiel 39 beschriebenen Arbeitsweise werden unter Verwendung von 8,41 g (50 mMol) 3,4-Dimethylbenzoylchlorid 3,5 g (53 % d. Th.) 3,4-Dimethylstyrol als farblose Flüssigkeit erhalten. Durch Ueberführen in das Bromderivat erhält man hellgelbe Kristalle mit einem Fp. unter 40 °C.

Beispiel 45

Analog der in Beispiel 39 beschriebenen Arbeitsweise werden unter Verwendung von 9,98 g (50 mMol) 2-Methyl-3-nitrobenzoylchlorid und 5,76 g (50 mMol) N-Aethylmorpholin 4,15 g (51 % d. Th.) 2-Methyl-3-nitrostyrol als hellbraune Flüssigkeit erhalten. Durch Ueberführen in das Bromderivat erhält man weisse Kristalle vom Fp. 56,7 °C.

Beispiel 46

Beispiel 45 wird wiederholt unter Verwendung von 9,29 g (50 mMol) 4-Nitrobenzoylchlorid. Nach dem Aufarbeiten erhält man 2,2 g (30 % d. Th.) 4-Nitrostyrol als gelbe Flüssigkeit. Das Ueberführen in das Bromderivat ergibt weisse Kristalle vom Fp. 74,8 °C.

Beispiel 47

Beispiel 45 wird wiederholt unter Verwendung von 8,44 g (50 mMol) 4-Formylbenzoylchlorid. Nach dem Aufarbeiten erhält man 0,7 g (11 % d. Th.) 4-Formylstyrol als tiefgelbe Flüssigkeit. Das Ueberführen in das Bromderivat ergibt weisse Kristalle vom Fp. 65,9 °C.

Beispiel 48

Zu 25 ml Toluol werden unter Argon 2,39 g (12,5 mMol) Zimtsäurenitril-4-carbonsäurechlorid, 1,69 g (12,5 mMol) N-Benzyldimethylamin und 0,028 0 g (0,125 mMol) Palladiumacetat gegeben. Das Reaktionsgemisch wird während 4 Stunden bei 140 °C unter einem Aethylendruck von 10 bar gerührt. Dann wird das Gemisch bei Raumtemperatur mit 100 ml 2N HCl und 100 ml 2N NaOH ausgeschüttelt und über Magnesiumsulfat getrocknet. Anschliessend wird die Lösung eingedampft und auf Kieselgel mit Dichlormethan als Laufmittel chromatographiert. Das so erhaltene gelbe Oel wird in kochendem n-Pentan gelöst. Bei − 20 °C werden 0,84 g (44 % d. Th.) 4-Vinylzimtsäurenitril in Form weisser Kristalle gebildet ; Fp. 45,8 °C.

Beispiel 49

Zu 25 ml Toluol werden unter Argon 1,743 g (6,25 mMol) Biphenyl-4,4′-dicarbonsäuredichlorid,

1,88 ml (12,5 mMol) N-Benzyldimethylamin und 0,028 0 g (0,125 mMol) Palladiumacetat gegeben. Das Gemisch wird 1,5 Stunden bei 115 °C unter einem Aethylendruck von 10 bar gerührt. Dann wird das Gemisch bei Raumtemperatur filtriert und mit 40 ml 2N HCl und 40 ml 2N NaOH ausgeschüttelt. Schliesslich wird mit Magnesiumsulfat getrocknet, eingedampft und einmal aus n-Hexan umkristallisiert. Man erhält 0,17 g (12 % d. Th.) 4,4'-Divinyl-biphenyl als weisse Kristalle ; Fp. > 300 °C (Zers.).

Beispiel 50

Zu 40 ml p-Xylol werden unter Argon 3,83 g (20 mMol) Zimtsäurenitril-4-carbonsäurechlorid, 2,70 g (20 mMol) N-Benzyldimethylamin und 0,044 8 g (0,2 mMol) Palladiumacetat gegeben. Aethylen wird durch das Gemisch geleitet, und das Gemisch wird 23 Stunden bei 130 °C gerührt. Danach wird das Reaktionsgemisch bei 130 °C mit 40 ml p-Xylol verdünnt und filtriert. Zum Filtrat werden 10 ml n-Hexan zugegeben. Nach dem Abkühlen auf Raumtemperatur erhält man 0,39 g (15 % d. Th.) 4,4'-Bis-(2-cyanovinyl)stilben vom Fp. 220 °C.

Anwendungsbeispiele

Beispiel I

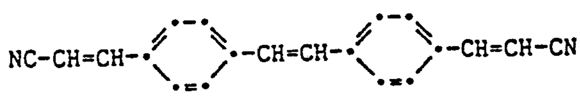

Zu einer Lösung von 9,85 mg (0,044 mMol) Palladiumacetat in 19,7 ml p-Xylol werden unter Argon 0,68 g (4,44 mMol) 4-Vinylzimtsäurenitril, 0,85 g (4,44 mMol) Zimtsäurenitril-4-carbonsäurechlorid und 0,60 g (4,44 mMol) N-Benzyldimethylamin gegeben. Das Gemisch wird während 4 Std. bei 130 °C gerührt, auf Raumtemperatur gekühlt und filtriert. Der Niederschlag wird mit Methanol nachgewaschen. Dann wird er in 20 ml heissem N,N-Dimethylformamid gelöst, mit 10 ml Wasser versetzt und auf 0 °C gekühlt. Man erhält 0,62 g (50 % d. Th.) des obigen optischen Aufhellers ; Fp. 220 °C.

Beispiel II

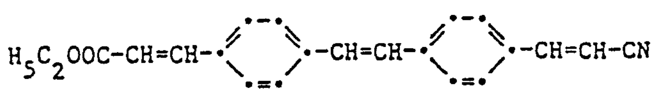

Zu 12,25 mg (0,054 9 mMol) Palladiumacetat in 24,5 ml p-Xylol werden unter Argon 0,84 g (5,49 mMol) 4-Vinylzimtsäurenitril, 1,309 g (5,49 mMol) Zimtsäureäthylester-4-carbonsäurechlorid und 0,742 g (5,49 mMol) N-Benzyldimethylamin gegeben. Das Gemisch wird während 5,5 Std. bei 130 °C gerührt. Dann wird das Gemisch mit 100 ml Toluol verdünnt und mit 100 ml 2N HCl und 100 ml 2N NaOH ausgeschüttelt. Nach dem Trocknen mit Magnesiumsulfat wird die Lösung eingedampft, und das Rohprodukt wird einmal aus 100 ml Tetrachlorkohlenstoff und einmal aus 50 ml Aethanol umkristallisiert. Man erhält 0,47 g (26 % d. Th.) des obigen optischen Aufhellers in Form hellgelber Kristalle ; Fp. 155,1 °C.

Beispiel III

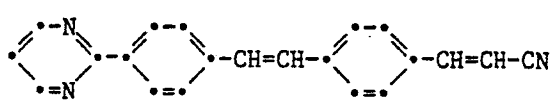

Zu 20 ml p-Xylol werden unter Argon 0,044 9 g (0,2 mMol) Palladiumacetat, 0,104 8 g (0,4 mMol) Triphenylphosphin, 4,7 g (20 mMol) 2-(4-Bromphenyl)-pyrimidin [hergestellt durch Umsetzung von 4-Brombenzamidin mit Malondialdehyd in basischem Milieu], 3,1 g (20 mMol) 4-Vinylzimtsäurenitril und 4,47 g (20 mMol) Tri-n-butylamin gegeben, und das Gemisch wird während 6 Std. bei 130° gerührt. Das erhaltene Rohprodukt wird bei Raumtemperatur abfiltriert und zweimal aus Toluol/Tetrachlorkohlenstoff umkristallisiert. Man erhält 1,9 g (31 % d. Th.) des obigen optischen Aufhellers in Form gelber Kristalle ; Fp. 290-291 °C.

**Patentansprüche**

1. Verfahren zur Herstellung von Verbindungen der Formel Ia oder Ib

$$Z{-}CH = CH{-}Z_1 \quad (Ia) \qquad oder \qquad CH_2 = CH{-}Z_2{-}CH = CH_2 \qquad (Ib),$$

worin

Z unsubstituiertes oder substituiertes Phenyl oder Naphthyl darstellt,

$Z_1$ Wasserstoff ist oder dieselbe Bedeutung wie Z hat und

$Z_2$ unsubstituiertes oder substituiertes Phenylen, Naphthylen oder p-Biphenylen oder einen unsubstituierten oder substituierten Stilbenrest bedeutet,

dadurch gekennzeichnet, dass man bei einem Druck von 0,1 bis 20 bar Aethylen in Gegenwart einer Base und unter Zusatz von Palladium-Metall oder von Palladiumverbindungen, die unter den Reaktionsbedingungen phosphorfreie labile Palladium (O) Verbindungen bilden, als Katalysator, mit einer Verbindung der Formel II oder III

$$Z\text{—}COX \quad (II) \qquad \text{oder} \qquad XOC\text{—}Z_2COX \quad (III)$$

umsetzt, wobei Z und $Z_2$ die unter Formel Ia bzw. Ib angegebenen Bedeutungen haben und X Chlor, Brom oder Jod darstellt.

2. Verfahren nach Anspruch 1, dadurch gekennzeichnet, dass man eine Verbindung der Formel II oder III verwendet, worin X Chlor darstellt.

3. Verfahren nach Anspruch 1, dadurch gekennzeichnet, dass man als Säurehalogenid Biphenyl-4,4'-dicarbonsäuredichlorid, Stilben-4,4'-dicarbonsäuredichlorid oder eine Verbindung der Formel IIa

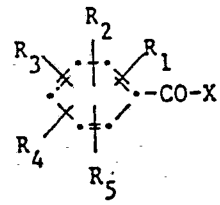

(IIa)

verwendet, worin X Chlor, $R_1$ Wasserstoff, Formyl, Methyl, Methoxy, Cl, Br oder Nitro, $R_2$ Wasserstoff, Methyl, Methoxy, Cl oder Br, $R_3$ Wasserstoff oder Methoxy und $R_4$ und $R_5$ Wasserstoff bedeuten, oder worin X Chlor darstellt, $R_1$—CH=CHCN, —CH=CHCOOCH$_3$ oder —CH=CHCOOC$_2$H$_5$ ist und $R_2$ bis $R_5$ Wasserstoff darstellen.

4. Verfahren nach Anspruch 1, dadurch gekennzeichnet, dass man als Katalysator $PdCl_2$, $PdBr_2$, $Pd(OOCCH_3)_2$, $Pd(CH_3COCHCOCH_3)_2$, $Pd(OOCCH_3)_2$(2,2'-Bipyridyl), $PdCl_2$(NC-Phenyl)$_2$, Bis-(Dibenzylidenaceton) Palladium(O) oder Bis-(Cyclohexylisonitril)-Palladium(O) verwendet.

5. Verfahren nach Anspruch 1, dadurch gekennzeichnet, dass man als Katalysator $PdCl_2$, Palladiumacetat oder Bis-(Benzylidenaceton) Palladium(O) verwendet.

6. Verfahren nach Anspruch 1, dadurch gekennzeichnet, dass man die Umsetzung mit den säurehalogeniden der Formel II bei einem Druck zwischen 0,1 und 1 bar durchführt.

7. Verfahren nach Anspruch 6, zur Herstellung von Verbindungen der Formel Ia, worin $Z_1$ unsubstituiertes Phenyl oder Naphthyl bedeutet, dadurch gekennzeichnet, dass die Umsetzung bei einem Druck von 1 bar durchgeführt wird.

8. Verfahren nach Anspruch 7, zur Herstellung der Verbindung der Formel

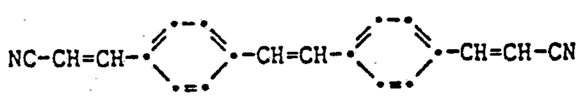

9. Verfahren nach Anspruch 7, zur Herstellung der Verbindung der Formel

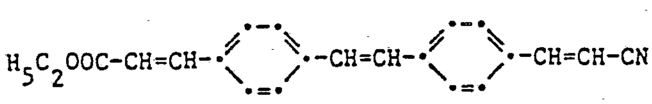

10. Verfahren nach Anspruch 1, dadurch gekennzeichnet, dass man die Umsetzung mit den Säurehalogeniden der Formel II oder III bei einem Druck zwischen 5 und 15 bar durchführt.

11. Verfahren nach Anspruch 10, zur Herstellung von Verbindungen der Formel Ia, worin $Z_1$ Wasserstoff bedeutet oder der Formel Ib, worin $Z_2$ unsubstituiertes oder substituiertes Phenylen, Naphthylen oder p-Biphenylen bedeutet, dadurch gekennzeichnet, dass die Umsetzung bei einem Druck von 10 bar durchgeführt wird.

12. Verfahren nach Anspruch 1, dadurch gekennzeichnet, dass man den Katalysator in einer Menge von 0,001 bis 3 Mol.%, bezogen auf die Verbindung der Formel II bzw. III, verwendet.

13. Verfahren nach Anspruch 1, dadurch gekennzeichnet, dass man die Umsetzung bei einer Temperatur zwischen 0 und 200 °C und in Gegenwart eines gegenüber den Reaktionspartnern inerten organischen Lösungsmittels vornimmt.

14. Verfahren nach Anspruch 13, dadurch gekennzeichnet, dass man als Lösungsmittel Anisol, Xylole oder Toluol verwendet.

15. Verfahren nach Anspruch 1, dadurch gekennzeichnet, dass man als Base eine Verbindung der Formel VI

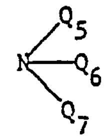

(VI)

verwendet, worin $Q_5$ 4-Chlorbenzyl, 4-Methylbenzyl, 4-Methoxybenzyl oder Benzyl und $Q_6$ und $Q_7$ je Alkyl mit 1-4 C-Atomen bedeuten, oder worin $Q_5$, $Q_6$ und $Q_7$ je Alkyl mit 3-12 C-Atomen bedeuten.

16. Verfahren nach Anspruch 1, dadurch gekennzeichnet, dass man als Base N-Benzyldimethylamin, N-Aethylmorpholin oder Tri-n-butyl-amin verwendet.

17. Die Verbindung der Formel

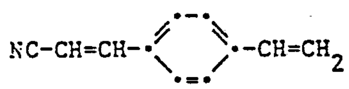

**Claims**

1. A process for the preparation of a compound of the formula Ia or Ib

$$Z{-}CH = CH{-}Z_1 \quad (Ia) \text{ or } CH_2 = CH{-}Z_2{-}CH = CH_2 \qquad (Ib)$$

in which

Z is unsubstituted or substituted phenyl or naphthyl,

$Z_1$ is hydrogen or has the same meaning as Z, and

$Z_2$ unsubstituted or substituted phenylene, naphthylene or p-biphenylene or an unsubstituted or substituted stilbene radical,

which process comprises reacting ethylene, under a pressure of 0.1 to 20 bar, in the presence of a base and with the addition, as catalyst, of a palladium metal or a palladium compound which forms a phosphorus-free labile palladium (O) compound under the reaction conditions, with a compound of the formula II or III

$$Z{-}COX \ (II) \quad \text{or} \quad XOC{-}Z_2COX \qquad (III)$$

in which Z and $Z_2$ are as defined for formulae Ia and Ib, and X is chlorine, bromine or iodine.

2. A process according to claim 1, which comprises the use of a compound of the formula II or III, in which X is chlorine.

3. A process according to claim 1, wherein the acid halide is biphenyl-4,4'-dicarboxylic acid dichloride, stilbene-4,4'-dicarboxylic acid dichloride or a compound of the formula IIa

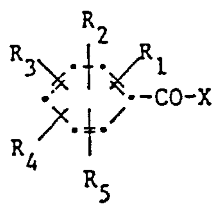

(IIa)

in which X is chlorine, $R_1$ is hydrogen, formyl, methyl, methoxy, Cl, Br or nitro, $R_2$ is hydrogen, methyl, methoxy, Cl or Br, $R_3$ is hydrogen or methoxy and $R_4$ and $R_5$ are hydrogen, or in which X is chlorine, $R_1$ is —CH=CHCN, —CH=CHCOOCH$_3$ or —CH=CHCOOC$_2$H$_5$ and $R_2$ to $R_5$ are hydrogen.

4. A process according to claim 1, wherein the catalyst is $PdCl_2$, $PdBr_2$, $Pd(OOCCH_3)_2$, $Pd(CH_3COCHCOCH_3)_2$, $Pd(OOCCH_3)_2$(2,2'-bipyridyl), $PdCl_2$(NC-phenyl)$_2$, bis-(dibenzylideneacetone) palladium(O) or bis(cyclohexylisonitrile) palladium(O).

5. A process according to claim 1, wherein the catalyst is $PdCl_2$, palladium acetate or bis(benzylidene-acetone) palladium(O).

6. A process according to Claim 1, wherein the reaction with the acid halide of the formula II is carried out under a pressure in the range from 0.1 to 1 bar.

7. A process according to claim 6 for the preparation of a compound of the formula Ia, in which $Z_1$ is unsubstituted phenyl or naphthyl, wherein the reaction is carried out under a pressure of 1 bar.

8. A process according to claim 7 for the preparation of the compound of the formula

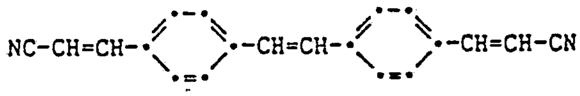

9. A process according to claim 7 for the preparation of the compound of the formula

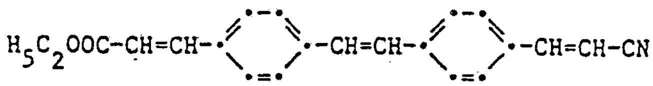

10. A process according to claim 1, wherein the reaction with the acid halide of the formula II or III is carried out under a pressure in the range from 5 to 15 bar.

11. A process according to claim 10 for the preparation of a compound of the formula Ia, in which $Z_1$ is hydrogen, or of the formula Ib, in which $Z_2$ is unsubstituted or substituted phenylene, naphthylene or p-biphenylene, wherein the reaction is carried out under a pressure of 10 bar.

12. A process according to claim 1, wherein the catalyst is used in an amount of 0.001 to 3 mol %, based on the compound of the formula II or III.

13. A process according to claim 1, wherein the reaction is carried out at a temperature in the range from 0 to 200 °C and in the presence of an organic solvent which is inert towards the reactants.

14. A process according to claim 13, wherein the solvent is anisole, a xylene or toluene.

15. A process according to claim 1, wherein the base is a compound of the formula VI

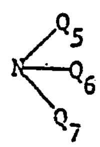

(VI)

in which $Q_5$ is 4-chlorobenzyl, 4-methylbenzyl 4-methoxybenzyl or benzyl and $Q_6$ and $Q_7$ are each alkyl having 1-4 carbon atoms, in which $Q_5$, $Q_6$ and $Q_7$ are each alkyl having 3-12 carbon atoms.

16. A process according to claim 1, wherein the base is N-benzyldimethylamine, N-ethylmorpholine or tri-n-butylamine.

17. The compound of the formula

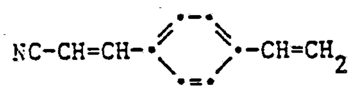

**Revendications**

1. Procédé de préparation de composés répondant à l'une des formules Ia et Ib

$$Z—CH = CH—Z_1 \quad (Ia) \quad et \quad CH_2 = CH—Z_2—CH = CH_2 \quad (Ib)$$

dans lesquelles

Z représente un radical phényle ou naphtyle, substitué ou non,

$Z_1$ représente l'hydrogène ou a la même signification que Z, et

$Z_2$ représente un radical phénylène, naphtylène ou p-biphénylylène, substitué ou non, ou encore un radical de stilbène substitué ou non,

procédé caractérisé en ce qu'on fait réagir l'éthylène, sous une pression de 0,1 à 20 bar, en présence d'une base et en ajoutant, comme catalyseur, du palladium métallique ou un composé du palladium formant, dans les conditions de la réaction, un composé du palladium (O) labile et dépourvu de phosphore, avec un composé répondant à l'une des formules II et III

$$Z—COX \ (II) \quad et \quad XOC—Z_2COX \quad (III)$$

dans lesquelles Z et $Z_2$ ont les significations données ci-dessus respectivement à propos des formules Ia et Ib, et X représente le chlore, le brome ou l'iode.

2. Procédé selon la revendication 1 caractérisé en ce qu'on utilise un composé de formule II ou III dans lequel X représente le chlore.

3. Procédé selon la revendication 1 caractérisé en ce qu'on utilise, comme halogénure d'acide, le dichlorure de l'acide biphényle-dicarboxylique-4,4', le dichlorure de l'acide stilbène-dicarboxylique-4,4' ou un composé répondant à la formule IIa

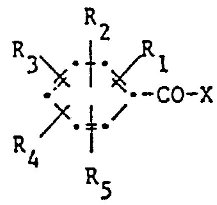

(IIa)

dans laquelle X représente le chlore, $R_1$ représente l'hydrogène, un formyle, un méthyle, un méthoxy, Cl, Br ou un nitro, $R_2$ représente l'hydrogène, un méthyle, un méthoxy, Cl ou Br, $R_3$ représente l'hydrogène ou un méthoxy, et $R_4$ et $R_5$ représentent chacun l'hydrogène, ou dans laquelle X représente le chlore, $R_1$ un radical $-CH=CHCN$, $-CH=CHCOOCH_3$ ou $-CH=CHCOOC_2H_5$, et $R_2$ à $R_5$ représentent chacun l'hydrogène.

4. Procédé selon la revendication 1 caractérisé en ce qu'on utilise, comme catalyseur, $PdCl_2$, $PdBr_2$, $Pd(OOCCH_3)_2$, $Pd(CH_3COCHCOCH_3)_2$, $Pd(OOCCH_3)_2$(bipyridyle-2,2'), $PdCl_2$(NC-phényl)$_2$, le bis(dibenzylidène-acétone)-palladium(O) ou le bis-(cyclohexylisonitrile)-palladium(O).

5. Procédé selon la revendication 1 caractérisé en ce qu'on utilise, comme catalyseur $PdCl_2$, l'acétate de palladium ou le bis-(benzylidène-acétone)-palladium(O).

6. Procédé selon la revendication 1 caractérisé en ce qu'on effectue la réaction avec les halogénures d'acides de formule II sous une pression comprise entre 0,1 et 1 bar.

7. Procédé selon la revendication 6, pour la préparation de composés de formule Ia dans lesquels $Z_1$ représente un radical phényle ou naphtyle non substitué, procédé caractérisé en ce qu'on effectue la réaction sous une pression de 1 bar.

8. Procédé selon la revendication 7 appliqué à la préparation du composé de formule

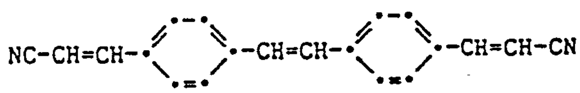

9. Procédé selon la revendication 7 appliqué à la préparation du composé de formule

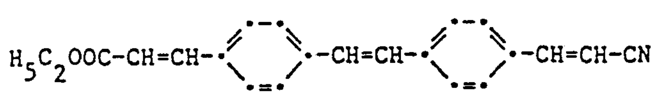

10. Procédé selon la revendication 1 caractérisé en ce qu'on effectue la réaction avec les halogénures d'acides de formule II ou III sous une pression comprise entre 5 et 15 bar.

11. Procédé selon la revendication 10 appliqué à la préparation de composés de formule Ia dans lesquels $Z_1$ représente l'hydrogène, ou de composés de formule Ib dans lesquels $Z_2$ représente un radical phénylène, naphthylène ou p-biphénylylène substitué ou non, procédé caractérisé en ce qu'on effectue la réaction sous une pression de 10 bar.

12. Procédé selon la revendication 1 caractérisé en ce qu'on utilse le catalyseur en une quantité de 0,001 à 3 % en moles par rapport au composé de formule II ou III.

13. Procédé selon la revendication 1 caractérisé en ce qu'on effectue la réaction à une température comprise entre 0 et 200 °C et en présence d'un solvant organique inerte à l'égard des corps prenant part à la réaction.

14. Procédé selon la revendication 13 caractérisé en ce qu'on utilise, comme solvants, l'anisole, les xylènes ou le toluène.

15. Procédé selon la revendication 1 caractérisé en ce qu'on utilise, comme base, un composé répondant à la formule VI

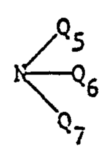

(VI)

dans laquelle $Q_5$ représente un radical chloro-4 benzyle, méthyl-4 benzyle, méthoxy-4 benzyle ou benzyle, tandis que $Q_6$ et $Q_7$ représentent chacun un alkyle contenant de 1 à 4 atomes de carbone, ou dans laquelle $Q_5$, $Q_6$ et $Q_7$ représentent chacun un alkyle contenant de 3 à 12 atomes de carbone.

16. Procédé selon la revendication 1 caractérisé en ce qu'on utilise, comme base, la N-benzyldiméthylamine, la N-éthyl-morpholine ou la tri-n-butylamine.

17. Composé de formule